(19) Europäisches Patentamt
European Patent Office
Office européen des brevets

(11) **EP 2 032 967 B1**

(12) **EUROPÄISCHE PATENTSCHRIFT**

(45) Veröffentlichungstag und Bekanntmachung des Hinweises auf die Patenterteilung:
**09.08.2017  Patentblatt 2017/32**

(51) Int Cl.:
*G01N 21/53* (2006.01)          *G01N 21/85* (2006.01)
*A61M 1/16* (2006.01)          *A61B 5/1455* (2006.01)

(21) Anmeldenummer: **07785849.6**

(22) Anmeldetag: **26.06.2007**

(86) Internationale Anmeldenummer:
**PCT/EP2007/005631**

(87) Internationale Veröffentlichungsnummer:
**WO 2008/000433 (03.01.2008 Gazette 2008/01)**

(54) **SPEKTROSKOPISCHER DETEKTOR UND VERFAHREN ZUR BESTIMMUNG VON BLUT UND BIOLOGISCHEN MARKERSUBSTANZEN IN FLÜSSIGKEITEN**

SPECTROSCOPIC DETECTOR AND METHOD FOR DETERMINING THE PRESENCE OF BLOOD AND BIOLOGICAL MARKER SUBSTANCES IN LIQUIDS

DÉTECTEUR SPECTROSCOPIQUE ET PROCÉDÉ POUR DÉTERMINER LA PRÉSENCE DE SANG ET DE MARQUEURS BIOLOGIQUES DANS DES LIQUIDES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **29.06.2006  DE 102006029899**

(43) Veröffentlichungstag der Anmeldung:
**11.03.2009  Patentblatt 2009/11**

(73) Patentinhaber: **Fresenius Medical Care Deutschland GmbH**
**61352 Bad Homburg v.d.H. (DE)**

(72) Erfinder:
• **BADO, Itka**
**60385 Frankfurt (DE)**
• **HERRENBAUER, Michael**
**61267 Neu Anspach (DE)**
• **MOISSL, Ulrich**
**61118 Bad Vilbl (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**EP-A- 0 575 712     EP-B1- 1 083 948**
**GB-A- 1 320 533     US-A- 5 385 539**
**US-A- 5 644 402     US-A- 5 783 826**
**US-B1- 6 806 947**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Detektor und die Detektion von Blut und biologischen Markersubstanzen in optisch dichten und klaren Flüssigkeiten oder in Sekundärflüssigkeiten, die in Blutreinigungsmaschinen verwendet werden.

Zur Gewährleistung der Patientensicherheit muss bei Einsatz eines Membranfilters zur Blutreinigung ein Blutdetektor eingesetzt werden, um patientenkritische Zustände durch Gefährdungen, wie einen möglichen Blutverlust, eine Membranruptur des Filters, ein Vertauschen von Anschlüssen oder einer Hämolyse zu vermeiden.

Aus dem Stand der Technik sind bereits spektroskopische Analysen zur Bestimmung von Blut und biologischen Markersubstanzen in Lösung bekannt.

So beschreibt die EP 0 575 712 A2 die spektroskopische Analyse von Blut an einem eingequetschen Schlauch in der Dialyse und die Messung in Transmission.

Die EP 1 083 948 B1 beschreibt die spektrale Messung von Abfallprodukten in Dialysierflüssigkeit, wobei die Messung direkt an der während der Dialysebehandlung austretenden Dialysierflüssigkeit vorgenommen wird. Die Messung wird spektralphotometrisch vorgenommen und der erhaltene Messwert mit der Durchflussmenge an Dialysierflüssigkeit multipliziert. Die Messung geschieht in Transmission.

In der US 5,644,402 ist ein optischer Detektor zur Bluterkennung in Blutbehandlungsgeräten offenbart, bei dem das Transmissionsverhalten der zu messenden Anordnung über ein mehrfaches Durchqueren des Messmediums gemessen wird.

In der US 6,718,190 B 1 ist eine Transmissionsanalyse mit geneigten optischen Achsen offenbart.

Die WO 2004/057313 beschreibt optische Messungen an einem eingequetschten Schlauch, bei dem verschiedene Lichtquellen und Sensoren und nicht geneigte optische Achsen verwendet werden.

Die US 5 783 826 A beschreibt eine Vorrichtung zur Phasenanalyse einer Mehrphasenmischung, beispielsweise zur Erfassung der Entmischung oder Sedimentation. Die Messvorrichtung beruht darauf, die Veränderungen der optischen Eigenschaften der Mehrphasenmischung an unterschiedlichen Stellen der Probe festzustellen. Es wird vorgeschlagen, sowohl die elektromagnetische Strahlung zu messen, die durch das Gemisch zurückgestreut wird, als auch die durch das Gemisch transmittierte elektromagne-tische Strahlung zu erfassen. Daher sind zwei Lichtdetektoren vorgesehen, von denen der eine Detektor dem Lichtemitter gegenüberliegt und der andere Detektor auf der gleichen Seite wie der Emitter angeordnet ist. Die Probe befindet sich in einem zylindrischen Körper, der entlang seiner Längsachse abgetastet wird. Der zylindrische Körper sitzt dabei mittig in der Messzelle der Abtastanordnung.

Aus der GB 1 320 533 A ist ein Verfahren zur Detektion von Blut in Lösung bekannt, bei dem aus einem wellenlängenabhängigen Messsignal und einem wellenlängenabhängigen Referenzsignal eine wellenlängenabhängige Signalveränderungsfunktion berechnet und auf das Vorhandensein von Blut geschlossen wird.

Optische Transmissionsmessungen sind jedoch insbesondere bei optisch dichteren Lösungen nicht möglich oder ungenau. Hier stellt sich die Frage, wie bestimmte Komponenten in der Flüssigkeit mit ausreichender Genauigkeit erfasst werden können. Auch kann es notwendig sein, Blut in Sekundärflüssigkeiten zu erkennen, um ein Blutleck in der Blutreinigungseinheit (z.B. Dialysator) feststellen zu können.

Aufgabe der Erfindung war demgemäß die Bereitstellung eines Detektors zur Detektion von Blut in einem über den Filter laufenden Sekundärkreislauf, in dem sich eine optisch dichte, suspendierte Lösung befindet. Gegebenenfalls sollte mit dem Detektor Blut, insbesondere auch in einer optisch klaren Lösung, detektiert werden können.

Zusätzlich bestand die Aufgabe, durch quantitative Bestimmung bestimmter Markersubstanzen (z.B. Bilirubin in der Leberunterstützungstherapie) den Therapiefortschritt zu kontrollieren und den Zeitpunkt des Behandlungsendes oder eines notwendigen Adsorbertausches bestimmen zu können. Der Detektor sollte auch die grundlegende Unterscheidung von optisch klarer und optisch dichter Lösung ermöglichen können.

Gelöst werden diese Aufgaben durch einen Detektor nach Anspruch 1.

In den Unteransprüchen zu Anspruch 1 sind besonders vorteilhafte Ausführungsformen des Detektors angegeben.

Ferner ist in Anspruch 13 eine Vorrichtung zur Blutbehandlung angegeben, die von dem Detektor Gebrauch macht.

Anspruch 14 betrifft ein Verfahren zur Detektion von Blut in Lösung, bei dem der Detektor verwendet wird, und Anspruch 15 betrifft die Verwendung des Detektors.

Bereitgestellt wird durch die Erfindung ein Detektor zur Messung von Streulicht in Flüssigkeiten, der ein Gehäuse, einen durch das Gehäuse geführten, lichtdurchlässigen, flexiblen, Flüssigkeit transportierenden Schlauch, einen Lichtemitter und einen Lichtdetektor umfasst, wobei in dem Gehäuse zwei im wesentlichen plane Flächen gebildet sind, zwischen denen der Schlauch derart angeordnet ist, dass zwei gegenüberliegende Schlauchwände im wesentlichen plan geformt sind, und der Lichtemitter mit seiner optischen Achse senkrecht zu den planen Flächen neben der ersten Schlauchwand angeordnet ist und der Lichtdetektor neben derselben ersten Schlauchwand wie der Lichtemitter, benachbart zu dem Lichtemitter, angeordnet ist, wobei die optischen Achsen von Lichtemitter und Lichtdetektor einen Winkel von kleiner als 90° bilden.

Gemäß einer bevorzugten Ausführungsform sind die planen Flächen der Schlauchwände parallel zueinander angeordnet.

Für den flexiblen, Flüssigkeit transportierenden Schlauch kann es im Rahmen der Erfindung ausreichend sein, wenn die an der planen Fläche angeordnete Schlauchwand flexibel ist, während die anderen Begrenzungswände rigide ausgestaltend sein können. Im allgemeinen Fall wird der Schlauch jedoch vollumfänglich flexibel sein.

Der Lichtemitter strahlt in einem breiten Wellenlängenbereich durch eine Blende Licht in einem senkrechten Winkel auf den Schlauch ein. Als Lichtquelle wird vorzugsweise eine weiße LED eingesetzt, die Wellenlängen im Bereich von ca. 400 bis 700nm emittiert. Der Schlauch ist vorzugsweise ein Standardschlauch, wie er in der Dialyse eingesetzt wird. Bevorzugt ist ein Einwegschlauch.

Dadurch, dass der Lichtemitter mit seiner optischen Achse senkrecht zu den planen Flächen neben der ersten Schlauchwand angeordnet ist, wird ein optimaler Durchtritt des Lichts in das Schlauchinnere ermöglicht.

Der erfindungsgemäße Detektor kann auch an optisch dichten Flüssigkeiten eingesetzt werden. Unter einer optisch dichten Flüssigkeit wird erfindungsgemäß eine lichtundurchlässige Flüssigkeit verstanden. Unter einer optisch klaren Flüssigkeit wird eine Flüssigkeit mit hoher Transmission für sichtbares Licht verstanden. Mit lichtdurchlässig ist gemeint, dass zumindest ein Teil des eingestrahlten Lichts den gesamten Schlauchquerschnitt samt der im Schlauch enthaltenen Flüssigkeit durchdringen kann, wenn das Licht auf einer Seite des Schlauches eingestrahlt wird. Bei lichtundurchlässigen Flüssigkeiten tritt praktisch kein Licht durch den Schlauchquerschnitt und die im Schlauch enthaltene Flüssigkeit, d.h. eine Transmission findet nicht statt.

Unter dem Oberbegriff "Flüssigkeit" werden erfindungsgemäß insbesondere Lösungen und Suspensionen verstanden.

In optisch dichter Flüssigkeit als erste Ausführungsform dringt das Licht durch die transparente Schlauchwand in die Flüssigkeit im Schlauchinneren ein und wird dort wellenlängenspezifisch teilweise absorbiert bzw. teilweise gestreut. Eine optisch dichte Flüssigkeit ist z.B. eine optisch dichte Suspension oder eine optisch dichte Lösung. Durch die optische Dichte der Flüssigkeit wird die Transmission durch das Flüssigkeitsinnere verhindert, so dass es auch zu keiner Reflektion an der vom Lichtemitter abgewandten rückwärtigen Schlauchwand oder an der rückwärtigen Gehäusewand, an der die rückwärtige Schlauchwand anliegt, kommt.

In optisch klarer Lösung als zweite Ausführungsform dringt das Licht ebenfalls durch die erste transparente Schlauchwand in die Flüssigkeit im Schlauchinneren ein. In diesem Fall durchdringt zumindest aber ein Teil des Lichts die im gesamten Schlauchquerschnitt befindliche Flüssigkeit im Schlauchinneren und wird bei einer bevorzugten Ausführungsform an der vom Lichtemitter abgewandten, rückwärtigen Schlauchwand und/oder der rückwärtigen Gehäusewand reflektiert. Die rückwärtige Gehäusewand ist die Wand im Inneren des Gehäuses, an der die rückwärtige Schlauchwand anliegt. Im Fall einer optisch klaren Lösung ist die reflektierte Lichtmenge aufgrund des zweimaligen Durchtritts durch die Flüssigkeit geringer als die eingestrahlte Lichtmenge.

Der Lichtdetektor befindet sich vorzugsweise mit seiner optischen Achse im 45° Winkel zur optischen Achse des Lichtemitters. Der Lichtdetektor nimmt das reflektierte bzw. gestreute Licht auf und wertet das Signal aus. Er umfasst vorzugsweise einen Lichtleiter, der das Licht aufnimmt, und ein Spektrometer, das mit dem Lichtleiter verbunden ist. Es wird z.B. das Licht über einen Lichtwellenleiter in ein Mikrospektrometer geleitet und dort das Wellenlängenspektrum aufgenommen.

Der Schnittpunkt der optischen Achsen von Lichtemitter und Lichtdetektor liegt in einer besonders vorteilhaften Ausführungsform genau an der Mediengrenze zwischen der dem Lichtemitter/Lichtdetektor zugewandten Schlauchwand und der im Schlauch transportierten Flüssigkeit.

Der Schnittpunkt kann aber je nach Dichte der Lösung auch wenige Zehntel Millimeter hinter der Mediengrenze im Schlauchinneren in der Flüssigkeit liegen. In einer weiteren vorteilhaften Ausführungsform liegt der Schnittpunkt der optischen Achsen von Lichtemitter und Lichtdetektor somit in einem Bereich, der sich von der Mediengrenze zwischen der dem Lichtemitter/Lichtdetektor zugewandten Schlauchwand und der im Schlauch transportierten Flüssigkeit bis hin zu 0,5 mm ins Schlauchinnere erstreckt.

Wie zuvor dargestellt, wird in optisch klarer Lösung Licht nach Durchdringen des gesamten Schlauchquerschnitts an der an der vom Lichtemitter abgewandten rückwärtigen Schlauchwand und/oder der rückwärtigen Gehäusewand reflektiert. Um in diesem Fall die Lichtreflexion zu verbessern, ist vorzugsweise an der vom Lichtemitter und Lichtdetektor abgewandten Seite des Schlauches eine reflektierende Oberfläche vorgesehen. Die reflektierende Oberfläche kann sowohl eine reflektierende Schlauchoberfläche als auch eine reflektierende Gehäusewand oder auch beides sein. Um eine Reflexion zu erzielen, bietet sich als Gehäusematerial z.B. Aluminium an. Ansonsten ist das Gehäuse vorzugsweise aus Kunststoff gefertigt. Zum einfachen Einlegen des Schlauches in das Gehäuse ist es von Vorteil, an der rückwärtigen, von Lichtemitter und -detektor abgewandten Seite einen Deckel vorzusehen.

Vorteilhafterweise ist zumindest eine der beiden Gehäusewände (die dem Lichtemitter zugewandte bzw. abgewandte rückwärtige Gehäusewand) plan ausgebildet, so dass sich die Schlauchwand beim Einlegen in das Gehäuse plan ausbildet. Gemäß einer bevorzugten Ausführungsform, die insbesondere bei doppelter Transmission zum Einsatz kommt, sind beide Gehäusewände plan und parallel zueinander ausgebildet, so dass die zu durchstrahlende Schichtdicke innerhalb des eingelegten Schlauchs im wesentlichen parallel und somit konstant ist.

Zwischen dem Schlauch und dem Lichtemitter und dem Lichtdetektor kann eine lichtdurchlässige Glasscheibe angeordnet sein, um eine Verschmutzung der Lichtemitter- bzw. Lichtdetektoröffnungen im Gehäuse zu vermeiden (z.B. bei einem Schlauchleck). Diese Glasscheibe kann vorzugsweise parallel zur gegenüberliegenden Deckeloberfläche angeordnet sein.

Vorzugsweise wird das Licht durch eine 2-4 mm breite Öffnung (Blende) in die Lösung gestrahlt, wodurch das System relativ unempfindlich gegen im Bereich der Fehlertoleranz liegende Abweichungen der Bohrungen bei der Gehäuseerstellung oder der Schlauchdicke wird.

Gelangt Hämoglobin an den Detektor, erhöht sich die Lichtabsorption und reduziert sich dementsprechend gleichzeitig die reflektierende Lichtmenge substanzspezifisch bei spezifischen Wellenlängen. Die Signaländerung im Vergleich zum vorher bestimmten Referenzsignal an diesen Wellenlängen, bei welchem die Lichtreflektion der puren Suspension bzw. Lösung gemessen wird, ergibt einen Signalausschlag für Blut, der unter Verwendung eines nachfolgend beschriebenen Algorithmus dann bei Erfüllen eines definierten Alarmkriteriums Blutalarm auslöst. Das gleiche Messsystem kann ebenfalls quantitativ für andere Substanzen verwendet werden, deren Spektralmaxima nicht durch Störsubstanzen überlagert werden.

Zum Zweck der Signalauswertung umfasst der Lichtdetektor vorteilhaft eine Auswertungseineinheit. Die Auswertungseinheit errechnet aus einem wellenlängenabhängigen Messsignal und einem ebenso wellenlängenabhängigen Referenzsignal eine wellenlängenabhängige Signalveränderung $\Delta$S gemäß der folgenden Formel (1):

$$S(\lambda) = \quad = \log\left(\frac{I(\lambda)_{referenz}}{I(\lambda)_{messwert}}\right) \qquad (1)$$

$$\lambda = \textit{Wellenlänge}, \; I = \textit{Intensität}$$

Anschließend bildet die Auswerteeinheit ein Faltungsintegral, beginnend von einer Wellenlänge $\lambda_0$ der Signalveränderungsfunktion $\Delta S(\lambda)$ über einen definierten Wellenlängenbereich z.B. von $\lambda_0$ bis $\lambda_1$. Die Faltungsfunktion lautet gemäß (2) z.B. folgendermaßen:

$$\psi(x) = \left(\frac{a}{\sqrt{b}}(\pi)^c\right)\cdot\left(d-\left(\frac{x}{j}\right)^f\right)\cdot e^{\frac{-\left(\frac{x}{j}\right)^g}{h}} \qquad (2),$$

wobei x = $\lambda$ - $\lambda_0$ und a, b, c, d, f, g, h und j gewählte Konstanten sind.

Für jede Wellenlänge wird die Signalveränderungsfunktion $\Delta$S mit der Faltungsfunktion $\psi$(x) multipliziert. Dann wird aus der Summe aller Produkte das Faltungsintegral für die Wellenlänge $\lambda_0$ errechnet. Befindet sich kein Blut im Medium, so sollte das Faltungsintegral unabhängig von allen Störeinflüssen möglichst bei Null liegen. Bei Blut wird es dagegen positiv. Der so gewonnene Wert wird mit verschiedenen definierten Alarmkriterien verglichen. Ist eines dieser Kriterien erfüllt, wird Blut erkannt.

Damit die Spektrendivergenz bei unterschiedlichen Sauerstoffsättigungen keinen Einfluss auf das Faltungsintegral hat, wird $\lambda_0$ (vorzugsweise $\lambda_0$ = 558nm) so gewählt, dass bei dieser Wellenlänge die Signalstärken von mit Sauerstoff gesättigtem und mit Sauerstoff ungesättigtem Blut gleich groß sind.

Bei der Auswertung können also zwei unterschiedliche Auswertungsmethoden zum Einsatz kommen. Bei der Erkennung von Stoffen, wie beispielsweise Bilirubin, wird das Signal $\Delta S(\lambda)$ bei einer bestimmten Wellenlänge gewählt. Um andererseits die Erkennung von Blut trotz verschiedener Sauerstoffsättigung zu ermöglichen, wird weiterhin ein Faltungsintegral herangezogen. Hierbei wird die Signalveränderungsfunktion $\Delta S(\lambda)$ für jede Wellenlänge mit einer vorgewählten Faltungsfunktion $\psi$(x) multipliziert, wobei das Ergebnis summiert wird.

Die erfindungsgemäße Vorrichtung kann auch zur Überprüfung der Funktionalität der Detektoreinrichtung eingesetzt werden, wobei das Vorliegen von Blut im Messobjekt simuliert wird.

Hierzu werden als Lichtemitter vorteilhafterweise sowohl eine weiß strahlende LED als auch eine grün strahlende LED eingesetzt. Es werden nacheinander zwei Messungen durchgeführt, wobei vorteilhafter weise im ersten Messschritt beide LED's strahlen, während im darauffolgenden Messschritt nur die weiße LED strahlt. Durch Ausschalten der grünen LED wird Blut im Schlauch simuliert, da im Schlauch vorliegendes Hämoglobin u.a. im grünen Wellenlängenbereich absorbiert, wodurch weniger Licht in diesem Wellenlängenbereich zum Detektor gelangt.

Zur Überprüfung der Funktionalität wird die Signaländerung gemäß Formel (1) in abgeänderter Form bestimmt, nämlich nach Formel (3)

$$\Delta S\left(\lambda\right) = \log\left(\frac{I(\lambda)_{LED\ wei\ß\&gr\ddot{u}n}}{I(\lambda)_{LED\ wei\ß}}\right) \qquad (3)$$

Für den Signalwert I $(\lambda)_{LED}$ weiß kann vorteilhaft auch ein hinterlegter Referenzwert verwendet werden.

Anschließend wird analog zur Spektrenbewertung für Blut die gleiche Funktion verwendet und der sich hieraus ergebende Signalwert wird mit einer Ansprechschwelle verglichen. Dabei soll bei funktionierendem Detektor der Signalwert stets höher als die Ansprechschwelle sein. Wenn dies nicht der Fall ist, deutet dies auf eine Fehlfunktion des Detektors hin.

Diese Simulation lässt sich im übrigen sowohl in einer optisch klaren als auch in einer optisch dichteren Lösung durchführen.

Gemäß einer weiteren Ausführungsform ist auch die Luft-Erkennung in einer optisch dichteren Lösung möglich. Gelangt nämlich Luft in den Sekundärkreislauf-Schlauch, so wird hierdurch die Intensität des gemessenen Spektrums verringert. Dies ist darauf zurückzuführen, dass Luft einer optisch transparenten Lösung sehr ähnlich ist, wobei aufgrund der Anordnung des optischen Systems wie bei der optisch transparenten Lösung nur ein Teil des reflektierten Lichts den Detektor erreicht.

In einem weiteren Aspekt betrifft die Erfindung ein Verfahren zur Detektion von Blut in Lösung, bei dem man die zuvor bei der Auswertungseinheit beschriebenen Verarbeitungsschritte durchführt. Die Detektion von Bilirubin mit dem erfindungsgemäßen Detektor ist besonders nützlich in der Leberunterstützungstherapie zur Kontrolle des Therapiefortschritts und Bestimmung des Zeitpunkts eines Behandlungsendes oder Adsorbertausches.

Demgemäss betrifft die Erfindung in einem weiteren Aspekt auch die Verwendung des erfindungsgemäßen Detektors in der Detektion von Blut, insbesondere Hämoglobin, und von biologischen Markersubstanzen, insbesondere Bilirubin.

Schließlich betrifft die Erfindung auch eine Vorrichtung zur Blutbehandlung mit einer Blutbehandlungseinheit, einem mit der Blutbehandlungseinheit verbundenen Blutkreislauf und einen ebenfalls mit der Blutbehandlungseinheit verbundenen Sekundärflüssigkeitskreislauf. Der Sekundärflüssigkeitskreislauf ist bevorzugt ein Dialysierflüssigkeitskreislauf. Der Sekundärflüssigkeitskreislauf umfasst ein Schlauchsystem und einen Detektor wie zuvor beschrieben, durch den der Schlauch des Sekundärflüssigkeitskreislaufs geführt ist.

Die Zeichnung erläutert die Erfindung.

Es zeigen:

Fig. 1 eine erste Ausführungsform des erfindungsgemäßen Detektors im Querschnitt, wobei hier eine Messung in optisch dichter Lösung dargestellt ist;

Fig. 2 die Ausführungsform des Detektors nach der Fig. 1 anhand einer Messung in optisch klarer Lösung;

Fig. 3a eine zweite Ausführungsform eines erfindungsgemäßen Detektors im Querschnitt anhand einer Messung in optisch dichter Lösung mit einem Lichtemitter und

Fig. 3b die gleiche Ausführungsform mit zwei Lichtemittern;

Fig. 4 einen Vergleich des Messsignals von optisch klarer und optisch dichter Lösung;

Fig. 5 Spektren des Hämoglobins für unterschiedliche Sauerstoffsättigungen;

Fig. 6 graphisch den Einfluss der zu messenden Konzentration an Hämoglobin auf das Messsignal;

Fig. 7 ein Spektrum des Bilirubins;

Fig. 8 den Einfluss der zu messenden Konzentration an Bilirubin auf das Messsignal;

Fig. 9 die Veränderung des Messsignals in optisch dichter Lösung ("ReferenzSignal") durch Zuführung von Hämoglobin ("Messsignal mit Hämoglobin");

Fig. 10 einen beispielhaften Flussplan einer Blutbehandlungsvorrichtung mit einem integrierten erfindungsgemäßen Detektor.

Die Erfindung wird nachfolgend detailliert mit Bezug auf bevorzugte Ausführungsformen beschrieben. Diese speziellen Ausführungsformen dienen nur zur Verdeutlichung und sollen die Erfindung, wie sie zuvor in allgemeiner Weise beschrieben wurde, nicht beschränken.

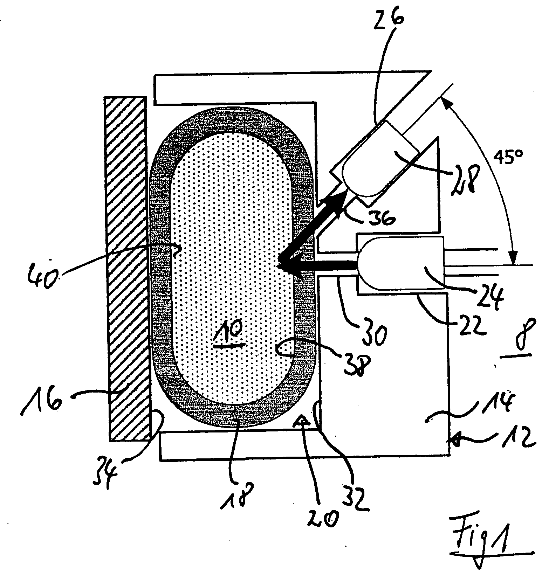

Fig. 1 zeigt eine erste Ausführungsform des erfindungsgemäßen Detektors 8 in einer Ansicht quer zur Fließrichtung der Flüssigkeit 10. Ein Gehäuse 12 ist gebildet aus einem Hauptgehäuseteil 14 und einem Gehäusedeckel 16, der zum einfachen Einlegen eines Schlauchs 18 in das Gehäuse 12 und als Messhintergrund dient. Bei eingelegtem Schlauch 18 ist der Gehäusedeckel 16 dicht abschließend auf dem Hauptgehäuseteil 14 befestigt.

Das Hauptgehäuseteil 14 umfasst weiterhin einen Kanal 20 zur Führung des Schlauches 18 durch das Gehäuse 12, eine erste Aussparung 22 zur Aufnahme eines Lichtemitters 24 sowie eine zweite Aussparung 26 zur Aufnahme eines Lichtdetektors 28. Beide Aussparungen 22 und 26 öffnen sich von der Außenseite des Hauptgehäuseteils 14 und gehen über eine verengte erste Blende 30 für den Strahlengang des Lichtemitters 24 und über eine zweite verengte Blende 36 für den Strahlengang des Lichtdetektors 28 durch das Hauptgehäuseteil 14 hindurch und münden im Kanal 20.

Der Lichtemitter 24 ist vorteilhafterweise eine weißes Licht aussendende Lichtquelle, vorzugsweise eine weißes Licht aussendende LED.

Gemäß einer weiteren Ausführungsform kann der Lichtemitter 24 im Bedarfsfall auch aus zwei Lichtquellen bestehen, die unterschiedliche Lichtspektren aussenden, beispielsweise einer weißes Licht aussendenden Lichtquelle und einer grünes Licht aussendenden Lichtquelle, insbesondere auch als LED-Ausführung.

Der Schlauch 18 ist durch das Gehäuse 12 geführt, wobei durch den Schlauch 18 Flüssigkeit 10 geleitet werden kann. Der Schlauch selbst ist durchlässig für das vom Lichtemitter 24 ausgesandte Licht.

Die plane Innenfläche 34 des Gehäusedeckels 16 ist vorteilhafterweise parallel zur gegenüberliegenden planen Kanalfläche 32 des Hauptgehäuseteils 14 angeordnet, wobei beide Flächen 32, 34 Begrenzungsflächen für den eingelegten Schlauch 18 sind. Dabei ist der Abstand der beiden Flächen 32 und 34 geringer als der Außendurchmesser des Schlauchs 18. Durch diese Anordnung ist der flexible Schlauch 18 nach Einlegen in das Gehäuse 12 so verformt, dass die zunächst runde Form in eine weitgehend ovale Form mit zwei nahezu planen, vorzugsweise planparallelen Schlauchwänden 32, 34 übergeht.

Der Lichtdetektor 28 ist benachbart zur ersten Kanalfläche 32 angeordnet, wobei auch hier über eine zweite Blende 36 zusammen mit der zweiten Aussparung 26 ein Lichtkanal durch das Hauptgehäuseteil 14 vom Kanal 20 her gebildet wird. Dieser Lichtdetektor 28 ist benachbart zum Lichtemitter 24 angeordnet, wobei die durch die beiden Blenden 30 und 36 sowie durch die ersten und zweiten Aussparungen 22 und 26 verlaufenden optischen Achsen vorteilhafterweise einen Winkel von etwa 45° bilden. In Fig. 1 sind die optischen Achsen, die zugleich auch die Lichtstrahlen anzeigen, von Lichtemitter 26 und Lichtdetektor 28 als Pfeile dargestellt, die sich an der Mediengrenze der ersten Schlauchwand 38 und der Flüssigkeit 10 oder wenige zehntel Millimeter hinter der Mediengrenze innerhalb der Flüssigkeit 10 schneiden.

Der Lichtdetektor 28 selbst ist gemäß seiner ersten Ausführungsform als Lichtleiter ausgebildet, der mit einem Spektrometer zur Weiterverarbeitung des Lichtsignals verbunden sein kann, oder aber als Lichtdiode u.dgl. gemäß einer zweiten Ausführungsform ausgebildet, die unmittelbar die eingestrahlte Lichtmenge in ein elektrisches Messsignal umwandeln kann.

In der Fig. 1 ist die Messung in optisch dichter Flüssigkeit dargestellt, wobei es ausreicht, wenn nur die erste Gehäusewand 32 plan ausgebildet wird, so dass gegebenenfalls eine plane Ausbildung der rückwärtigen Gehäusewand 34 nicht notwendig ist. Die Flüssigkeit 10 ist hier beispielsweise eine optisch dichte Lösung öder Suspension. Das Licht dringt durch die erste Schlauchwand 38 in die Lösung 10 ein und wird dort in der optisch dichten Flüssigkeit 10 wellenlängenspezifisch teilweise absorbiert und teilweise gestreut. Durch die optische Dichte in der Flüssigkeit 10 wird jegliche Transmission durch die Flüssigkeit 10 verhindert, so dass es zu keiner Reflektion an der zweiten Schlauchwand 40 oder an der Innenfläche 34 des Deckels 16, an der die zweite Schlauchwand 40 anliegt, kommt. Schematisch dargestellt ist hier lediglich die teilweise Streuung an der Flüssigkeitsoberfläche in Richtung des Lichtdetektors 28 in Form eines Pfeils zum Lichtdetektor 28.

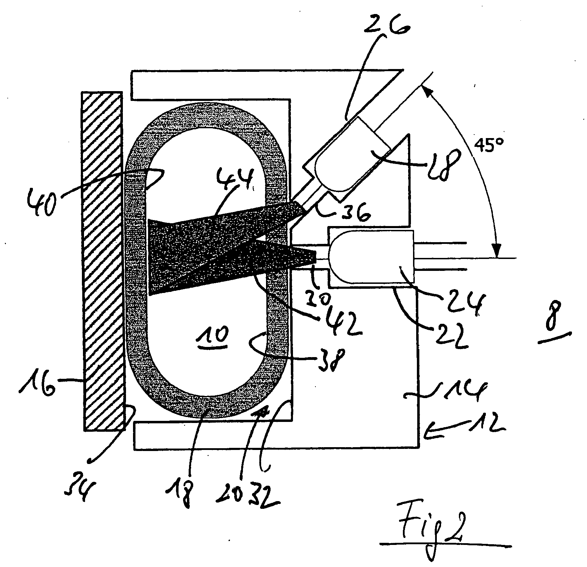

Fig. 2 zeigt die Ausführungsform des Detektors nach der Fig. 1 - mit dem Unterschied, dass hier eine Messung in optisch klarer Lösung dargestellt ist. Das vom Lichtemitter 24 ausgesendete Licht 42 durchdringt die erste Schlauchwand 38 und die gesamte, im Schlauch befindliche Flüssigkeit 10, um an der zweiten, rückwärtigen Schlauchwand 40 und/oder an der Innenfläche des Deckels 16 reflektiert zu werden. Ein Teil 44 des reflektierenden Lichts durchdringt wiederum die gesamte Flüssigkeit, um vom Lichtdetektor bzw. Lichtleiter 28 aufgenommen zu werden. Gemäß dieser Ausführungsform ist es vorteilhaft, wenn die rückwärtige Gehäusewand 34 planparallel zur vorderen ersten Gehäusewand 32 ausgebildet ist.

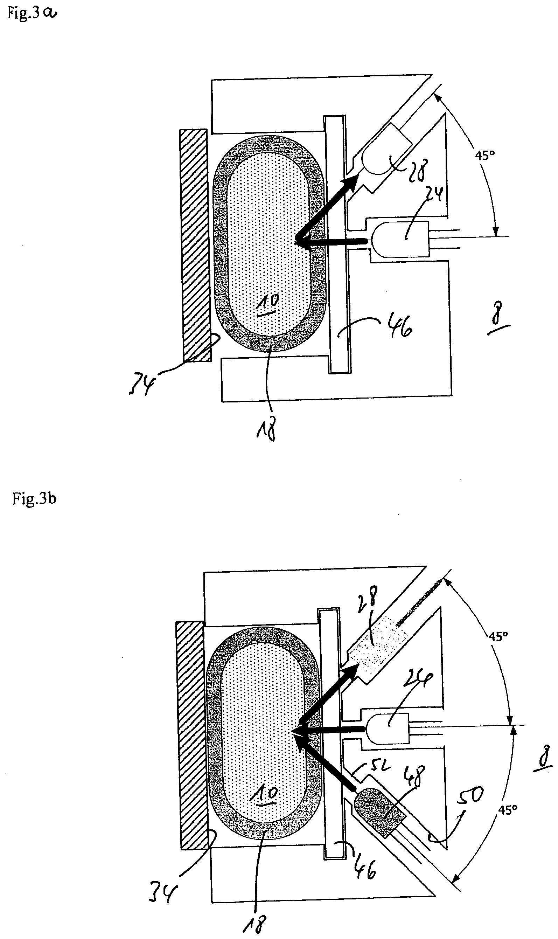

Fig. 3a zeigt eine zweite Ausführungsform des Detektors 8 im Querschnitt anhand einer Messung in optisch dichter Lösung. Im Unterschied zu der in den Fig. 1 und 2 gezeigten Ausführungsform ist zwischen der ersten Schlauchwand 38 und der Wandfläche eine Glasscheibe 46 angeordnet, die für das eingestrahlte Licht durchlässig ist. Dadurch werden mögliche Verschmutzungen der Lichtemitter- bzw. Lichtdetektoröffnungen im Gehäuse 12 vermieden. Vorteilhafterweise ist die rückwärtige Gehäusewand 34 planparallel zur Glasscheibe 46 ausgebildet.

Fig. 3b zeigt eine weitere Ausführungsform mit 2 Lichtemittern unterschiedlicher Einstrahlungswellenlänge (z.B. grün). Zum ersten Lichtemitter 24 kommt ein zweiter Lichtemitter 48 hinzu, der in einer dritten Aussparung 50 im Hauptgehäuseteil 14 aufgenommen ist. Die Aussparung 50 öffnet sich von der Außenseite des Hauptgehäuseteils 14 und geht über eine weitere verengte dritte Blende 52 für den Strahlengang des Lichtemitters 48 durch das Hauptgehäuseteil 14 hindurch und mündet ebenfalls im Kanal 20. Der Lichtstrahl bzw. die optische Achse des zweiten Lichtemitters 48 bildet - wie durch die Pfeile in Fig. 3a angedeutet - vorteilhafterweise einen Winkel von 45° zur Achse des ersten Lichtemitters 24, wobei auch andere Winkel möglich sind und lediglich von der Messgeometrie der eingesetzten Lichtemitter bzw. -detektoren abhängen. Die beiden Achsen schneiden sich an der Mediengrenze der ersten Schlauchwand 38 und der Flüssigkeit 10 oder wenige zehntel Millimeter hinter der Mediengrenze innerhalb der Flüssigkeit 10.

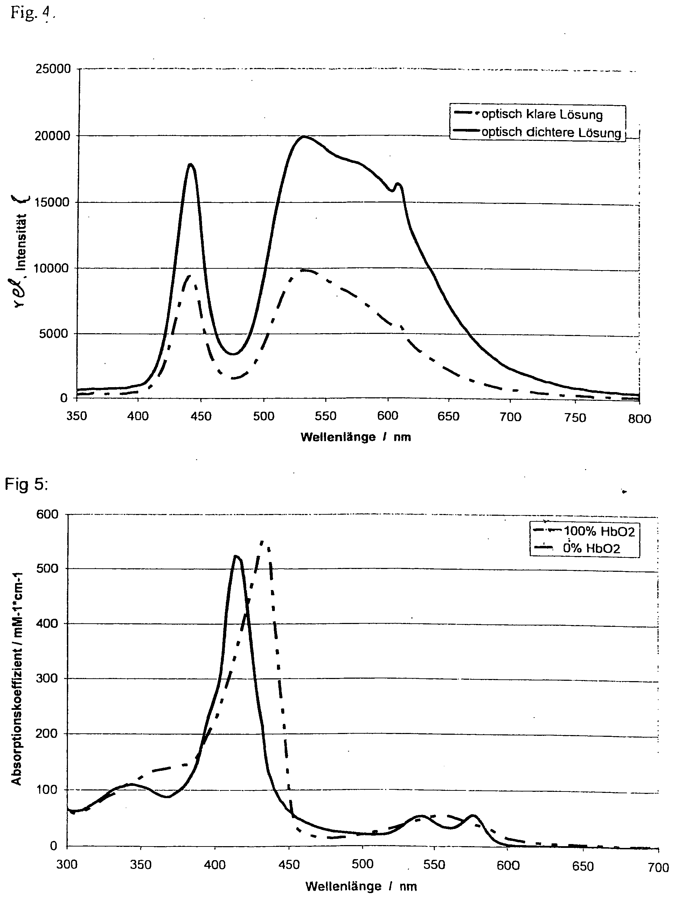

Fig. 4 zeigt einen Vergleich des Messsignals von optisch klarer und optisch dichter Lösung. Es ist dargestellt, wie bei gleichen Messparametern durch Veränderung der Messsignale eine optisch klare Lösung von einer optisch dichten Lösung unterschieden werden kann. Ist eine optisch dichte Lösung im Schlauch 18, so gelangt ein hoher Anteil des in das Medium eingestrahlten Lichtes über Streuung in den Lichtdetektor 28 und das Spektrometer. Bei optisch klarer Lösung wird das Licht an der hinteren Schlauchwand 40 oder an der Innenfläche des Deckels 16 reflektiert. Da die optischen Achsen sich hier aber nicht treffen, gelangt nur ein Teil des eingestrahlten Lichts in den Lichtdetektor 28. Die Gesamtlichtmenge ist daher bei klarem Medium deutlich niedriger (s. Fig. 4). Dies ermöglicht eine einfache und sichere Unterscheidung der verschiedenen Flüssigkeitszustände.

Fig. 5 zeigt Spektren des Hämoglobins für unterschiedliche Sauerstoffsättigungen. Der Farbstoff der Erythrozyten, das Hämoglobin, hat bei unterschiedlichen Sauerstoffsättigungen leicht divergierende Blutspektren. Diese Divergenz der Spektren kann bei der Auswertung durch den Algorithmus, wie nachfolgend gezeigt, ausgeglichen werden, um eine genaue quantitative Messung sicher zu stellen.

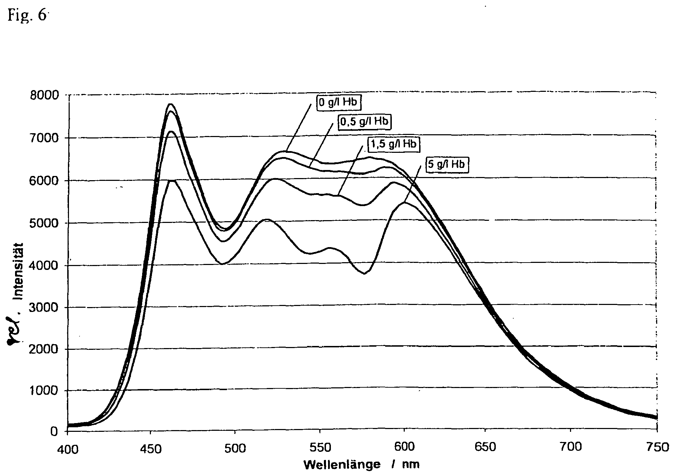

Fig. 6 stellt den Einfluss der zu messenden Konzentration an Hämoglobin auf das Messsignal dar. Durch Zugabe von Hämoglobin in eine optisch dichte Lösung reduziert sich das zurück gestreute Licht im Bereich der für die Substanz spezifischen Wellenlängen, wie in Fig. 6 dargestellt. Die Veränderung des Spektrums bei optisch klarer Lösung ist hierzu analog. Einzig das Gesamtsignal vor Hämoglobin-Zugabe unterscheidet sich, wie in Fig. 4 dargestellt, deutlich voneinander. Besonders im Bereich 500-600 nm ist die Spektrenänderung durch das Absorptionsverhalten sehr spezifisch. Dieser Bereich wird u.a. bei dem Auswertealgorithmus zur Bluterkennung angewendet. Überlagerungen in diesem Wellenbereich durch Markersubstanzen sind bei der Entwicklung des Auswertealgorithmus zu beachten. Der Hämoglobinpeak zwischen 400 und 450 nm kann für die Auswertung nicht herangezogen werden, da dieser Peak von Bilirubin, das in diesem Beispiel als Markersubstanz in der Flüssigkeit 10 enthalten sein kann, deutlich überlagert wird.

Die quantitative Bestimmung von Bilirubin erfolgt durch Auswertung des Peaks bei etwa 450 nm.

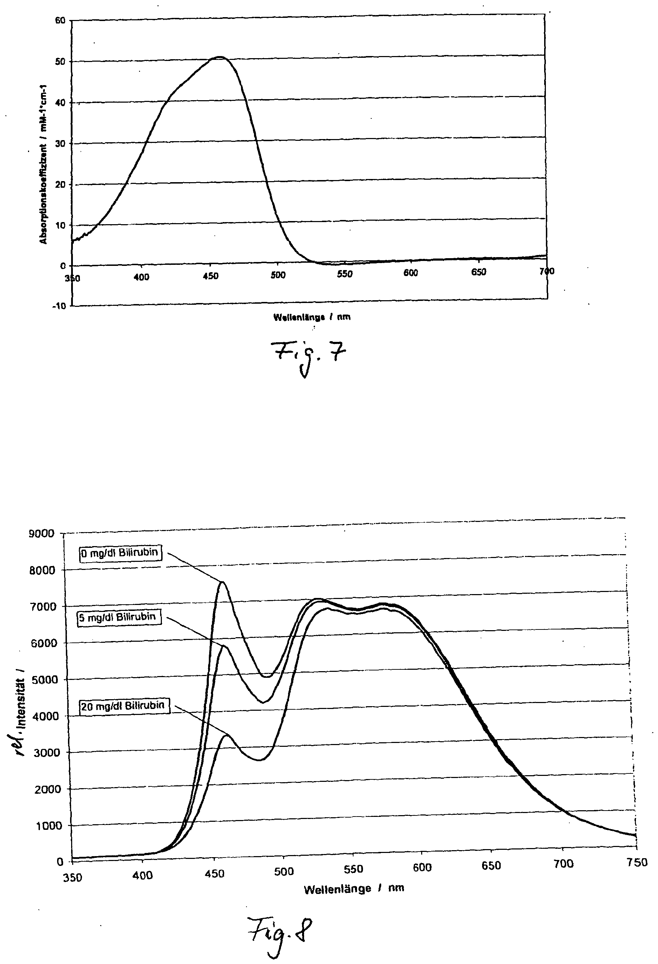

Fig. 7 zeigt ein Spektrum des Bilirubins.

In Fig. 8 ist die Änderung des Mess- bzw. Rohsignals durch unterschiedlich hohe Zugabe von Bilirubin zu erkennen.

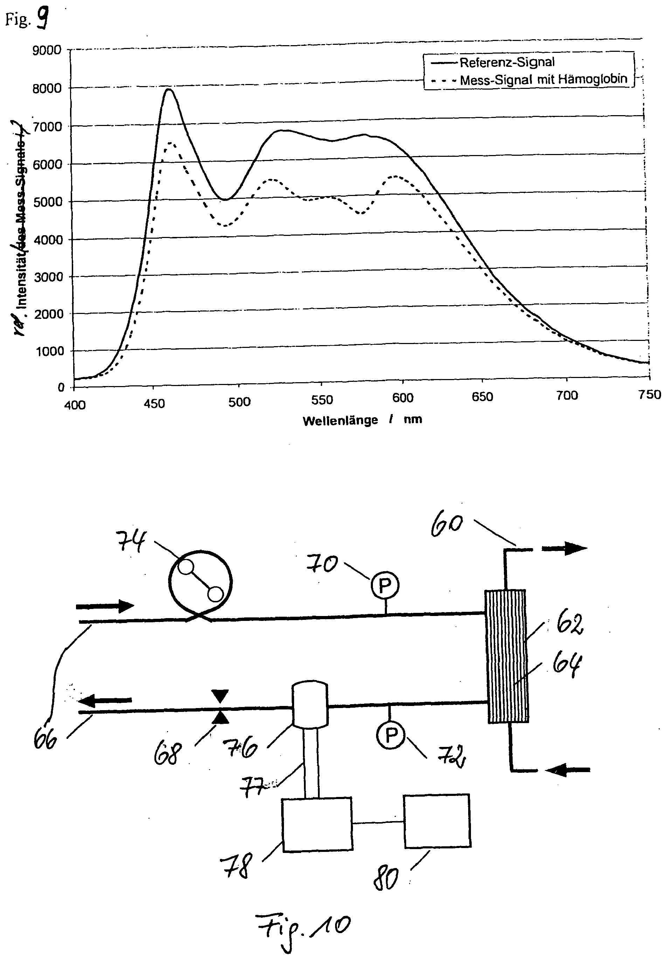

Störeinflüsse bei der Auswertung bezüglich von Hämoglobin kommen, wie zuvor erwähnt, von durch die Blutreinigung in die Flüssigkeit gelangte Substanzen wie z.B. Bilirubin (s. Fig. 7-8) sowie von der Spektrendivergenz des Blutes bei unterschiedlicher Sauerstoffsättigung (s. Fig. 5). Die Auswertung ist daher so ausgelegt, dass diese Störeinflüsse minimiert werden. Da die Lichtquelle im Detektor auch zur Messung des Referenzsignals eingesetzt wird, wird die Referenz zu einem Zeitpunkt aufgenommen, in dem der Schlauch eingelegt ist und sich eine Blindlösung im Schlauch befindet. Bei optisch klarer Lösung ist dies Wasser oder eine Kochsalzlösung. Bei einer optisch dichten Lösung sind zusätzlich Partikel enthalten. Die Fig. 9 zeigt ein Spektrum des Referenzsignals und ein Messspektrum mit Hämoglobin in optisch dichter Lösung.

Um Signalveränderungen bezogen auf das Referenzsignal darzustellen, werden die Daten des Referenzspektrums und des Messspektrums nach Formel (1) ausgewertet.

Um einen sicheren Nachweis von Blut liefern zu können, wird eine Faltungsfunktion verwendet, die z.B. die Form von Gleichung (2) aufweisen kann.

Um den Störeinfluss von Bilirubin zu minimieren, hat es sich als ausreichend erwiesen, die Faltungsfunktion in einem Wellenlängenbereich von ca. 550-700 nm heranzuziehen. Zur Bildung des Faltungsintegrals wird die Signalveränderungsfunktion $\Delta S(\lambda)$ mit der Faltungsfunktion $\psi(x)$ für jede Wellenlänge multipliziert. Die Summe der Produkte stellt dann das Faltungsintegral für die Wellenlänge $\lambda_0$ dar. Befindet sich kein Blut im Medium, so sollte das Faltungsintegral unabhängig von allen Störeinflüssen möglichst bei Null liegen. Bei Anwesenheit von Blut wird es dagegen positiv.

Um dies zu erreichen, ist $\lambda_0$ für eine bestimmte Faltungsfunktion entsprechend zu wählen, d.h. es sollte für gesättigtes und ungesättigtes Blut an dieser Wellenlänge gleich groß sein.

Fig. 10 zeigt einen Flussplan einer Blutbehandlungsvorrichtung 58 mit einem integrierten Detektor 76, der dem Detektor 8 gemäß den Ausführungsformen von Fig. 1 bis 3 entspricht.

Dargestellt ist ein Blutkreislauf 60 von und zu einem Patienten sowie ein Sekundärkreislauf 66 zur Blutreinigung.

Beide Kreisläufe 60 und 66 sind an eine Blutbehandlungseinheit 62 angeschlossen, wobei diese Kreisläufe innerhalb der Blutbehandlungseinheit 62 durch eine Membran 64 getrennt sind.

In dem Sekundärkreislauf 66, in dem üblicherweise eine Reinigungsflüssigkeit, z.B. eine Dialysierflüsskeit, umgepumpt wird, befinden sich die üblichen Klemmen 68, Drucksensoren 70 und 72 stromauf bzw. stromab der Blutbehandlungseinheit 62 sowie eine stromauf der Blutbehandlungseinheit 62 eingeschaltete Schlauchpumpe 74.

Der Detektor 76 ist stromab der Blutbehandlungseinheit 62 in den Sekundärkreislauf 66 eingeschaltet, wobei der Schlauch des Sekundärkreislaufs 66 den Schlauch 18 des Detektor 8 gemäß Fig. 1 - 3 bildet.

Wie aus Fig. 10 ersichtlich ist, ist an die Detektoreinheit 76 über einen Lichtleiter 77 ein Spektrometer 78 oder ein anderer Detektor angeschlossen, in dem die zugeführten Lichtsignale spektral zerlegt werden.

Eine an das Spektrometer 78 angeschlossene Auswerteeinheit 80 wertet dann das vom Spektrometer 78 kommende Signal aus, vergleicht das aktuelle Signal gegebenenfalls mit einem Referenzsignal und erzeugt über eine nicht gezeigte Rechen- und Diskriminiereinheit das Endsignal.

Dieses Endsignal kann anschließend zur nicht gezeigten Hauptsteuerung der Blutbehandlungsvorrichtung 58 zugeführt werden, um dort in einer Alarmeinheit weiter verarbeitet zu werden.

## Patentansprüche

1. Detektor zur Messung von Streulicht in Flüssigkeiten mit einem Gehäuse (12), das einen durch das Gehäuse (12) geführten, lichtdurchlässigen, flexiblen, Flüssigkeit (10) transportierenden Schlauch (18), einen Lichtemitter (24) und einen Lichtdetektor (28) umfasst,
   **dadurch gekennzeichnet, dass**
   in dem Gehäuse (12) wenigstens eine plane Fläche (32, 34) gebildet ist, an der der Schlauch (18) derart angeordnet ist, dass die anliegende Schlauchwand (31) plan geformt ist,
   und der Lichtemitter (24) mit seiner optischen Achse im wesentlichen senkrecht zu der planen Fläche an der Schlauchwand (38) angeordnet ist und
   der Lichtdetektor (28) an der Schlauchwand (38) benachbart zu dem Lichtemitter (24) angeordnet ist, wobei die optischen Achsen von Lichtemitter (24) und Lichtdetektor (28) einen Winkel von kleiner als 90° bilden.

2. Detektor nach Anspruch 1, **dadurch gekennzeichnet, dass** zwei Flächen (32, 34) bzw. die Schlauchwände (38, 40) planparallel angeordnet sind.

3. Detektor nach Anspruch 1, **dadurch gekennzeichnet, dass** die optischen Achsen von Lichtemitter (24) und Lichtdetektor (28) einen Winkel von 35 bis 55°, vorzugsweise etwa 45° bilden.

4. Detektor nach einem der Ansprüche 1 bis 3, **dadurch gekennzeichnet, dass** der Schnittpunkt der optischen Achsen von Lichtemitter (24) und Lichtdetektor (28) in einem Bereich liegt, der sich von der Mediengrenze zwischen der ersten Schlauchwand (38) und der im Schlauch (18) transportierten Flüssigkeit (10) bis hin zu 0,5 mm in die Flüssigkeit (10) erstreckt.

5. Detektor nach einem der Ansprüche 1 bis 3, **dadurch gekennzeichnet, dass** der Schnittpunkt der optischen Achsen von Lichtemitter (24) und Lichtdetektor (28) an der Mediengrenze zwischen der ersten Schlauchwand (38) und der im Schlauch transportierten Flüssigkeit (10) liegt.

6. Detektor nach einem der Ansprüche 1 bis 4, **dadurch gekennzeichnet, dass** an der von dem Lichtemitter (24) und dem Lichtdetektor (26) abgewandten Seite (34, 40) des Schlauches (10) eine reflektierende Oberfläche vorgesehen ist.

7. Detektor nach Anspruch 6, **dadurch gekennzeichnet, dass** die reflektierende Oberfläche eine reflektierende Schlauchoberfläche (40) oder eine reflektierende Gehäuseoberfläche (34) ist.

8. Detektor nach einem der Ansprüche 1 bis 7, **dadurch gekennzeichnet, dass** zwischen dem Schlauch (18) und dem Lichtemitter (24) und dem Lichtdetektor (26) eine lichtdurchlässige Glasscheibe (46) angeordnet ist.

9. Detektor nach einem Ansprüche 1 bis 7, **dadurch gekennzeichnet, dass** der Lichtemitter (24) Licht der Wellenlänge von 400 bis 700 nm aussendet.

10. Detektor nach einem der Ansprüche 1 bis 8, **dadurch gekennzeichnet, dass** der Lichtdetektor (26) ein Lichtleiter oder eine Lichtdiode ist.

**11.** Detektor nach einem der Ansprüche 1 bis 10, **dadurch gekennzeichnet, dass** der Lichtdetektor (26) ein mit dem Lichtleiter verbundenes Spektrometer umfasst.

**12.** Detektor nach einem der Ansprüche 1 bis 11, **dadurch gekennzeichnet, dass** der Lichtdetektor (26) weiterhin mit einer Auswertungseinheit (70) zum Blutnachweis verbunden ist, wobei die Auswertungseinheit (70) derart ausgebildet ist, dass die Auswertungseinheit aus einem wellenlängenabhängigen Messsignal und einem ebenso wellenlängenabhängigen Referenzsignal eine wellenlängenabhängige Signalveränderungsfunktion errechnet, ein Faltungsintegral aus der Signalveränderungsfunktion über einen definierten Wellenlängenbereich bildet, und aufgrund des Wertes des Faltungsintegrals auf das Vorhandensein von Blut in der Flüssigkeit schließt.

**13.** Vorrichtung zur Blutbehandlung mit einer Blutbehandlungseinheit (62), einem mit der Blutbehandlungseinheit (62) verbundenen Blutkreislauf (60) und einen ebenfalls mit der Blutbehandlungseinheit (62) verbundenen Sekundärkreislauf (66), insbesondere Dialysierflüssigkeitskreislauf, wobei der Sekundärkreislauf (66) ein Schlauchsystem und einen Detektor (8) nach den Ansprüchen 1 bis 12 umfasst.

**14.** Verfahren zur Detektion von Blut in Lösung, bei dem ein Detektor nach einem der Ansprüche 1 bis 12 verwendet wird, wobei aus einem wellenlängenabhängigen Messsignal und einem ebenso wellenlängenabhängigen Referenzsignal eine wellenlängenabhängige Signalveränderungsfunktion berechnet wird, und wobei ein Faltungsintegral aus der Signalveränderungsfunktion und einer Faltungsfunktion über einen definierten Wellenlängenbereich gebildet wird und aufgrund des Wertes des Faltungsintegrals auf das Vorhandensein von Blut geschlossen wird.

**15.** Verwendung des Detektors (8) nach den Ansprüchen 1 bis 12 zur Detektion von Hämoglobin und oder Markersubstanzen, insbesondere Bilirubin in Flüssigkeiten.

**16.** Verwendung des Detektors (8) nach den Ansprüchen 1 bis 12 zur Detektion von Lufteinschlüssen in einer optisch dichten Lösung.

## Claims

**1.** Detector for measuring stray light in liquids with a casing (12) comprising a light-transmissive flexible tube (18) conducted through the casing (12) and transporting liquid (10), a light emitter (24) and a light detector (28), **characterised in that**
in the casing (12), at least one flat surface (32, 34) is formed, on which the tube (18) is arranged such that the contiguous tube wall (31) is flat,
and the light emitter (24) is arranged with its optical axis being substantially vertical to the flat surface on the tube wall (38), and the light detector (28) is arranged on the tube wall (38) adjacent to the light emitter (24), whereby the optical axes of the light emitter (24) and light detector (28) form an angle smaller than 90°.

**2.** Detector according to claim 1, **characterised in that** the two surfaces (32, 34) of the tube walls (38, 40) are arranged to be plan parallel.

**3.** Detector according to claim 1, **characterised in that** the optical axes of the light emitter (24) and light detector (28) form an angle of 35 to 55°, preferably app. 45°.

**4.** Detector according to one of claims 1 to 3, **characterised in that** the intersection of the optical axes of the light emitter (24) and light detector (28) is located in an area extending from the media boundary between the first tube wall (38) and the liquid (10) transported in the tube (18) up to 0.5 mm into the liquid (10).

**5.** Detector according to one of claims 1 to 3, **characterised in that** the intersection of the optical axes of the light emitter (24) and light detector (28) lies on the media boundary between the first tube wall (38) and the liquid (10) transported in the tube.

**6.** Detector according to one of claims 1 to 4, **characterised in that** a reflecting surface is provided on the side (34, 40) of the tube (10) facing away from the light emitter (24) and the light detector (28).

7. Detector according to claim 6, **characterised in that** the reflecting surface is a reflecting tube surface (40) or a reflecting casing surface (34).

8. Detector according to one of claim 1 to 7, **characterised in that** a light-transmissive glass pane (46) is disposed between the tube (18) and the light emitter (24) and the light detector (28).

9. Detector according to one of claims 1 to 7, **characterised in that** the light emitter (24) emits light with the wavelength from 400 to 700 nm.

10. Detector according to one of claims 1 to 8, **characterised in that** the light detector (28) is a light conductor or light diode.

11. Detector according to one of claims 1 to 10, **characterised in that** the light detector (28) comprises a spectrometer connected with the light conductor.

12. Detector according to one of claims 1 to 11, **characterised in that** the light detector (28) is further connected with an analysis unit (70) for blood determination, which is configured to calculate from a wavelength-dependent measuring signal and a likewise wavelength-dependent reference signal a wavelength-dependent signal change function, forms a convolution integral from the signal change function for a defined wavelength range and determines the presence of blood in the liquid on the basis of the value of the convolution integral.

13. Apparatus for blood treatment comprising a blood treatment unit (62), a blood circuit (60) connected with the blood treatment unit (62) and a secondary circuit (66), in particular a dialysis liquid circuit, likewise connected with the blood treatment unit (62), whereby the secondary circuit (66) comprises a tube system and a detector (8) according to claims 1 to 12.

14. Process for the detection of blood in solution using a detector according to one of claims 1 to 12, whereby a wavelength-dependent signal change function is calculated from a wavelength-dependent measuring signal and a likewise wavelength-dependent reference signal,
a convolution integral is formed from the signal change function and a convolution function for a defined wavelength range and the presence of blood is determined on the basis of the value of the convolution integral.

15. Use of the detector (8) according to one of claims 1 to 12 for the detection of haemoglobin and/or marker substances, in particular bilirubin, in liquids.

16. Use of the detector (8) according to one of claims 1 to 12 for the detection of air occlusions in an optically dense solution.

**Revendications**

1. Détecteur pour mesurer la lumière diffusée dans des liquides avec un boîtier (12), qui comprend un tuyau (18) flexible, translucide, guidé à travers le boîtier (12) et transportant un liquide (10), un émetteur de lumière (24) et un détecteur de lumière (28),
   **caractérisé en ce que**
   au moins une surface plane (32, 34) est formée dans le boîtier (12), au niveau de laquelle le tuyau (18) est agencé de telle sorte que la paroi de tuyau (31) appliquée est formée de manière plane,
   et l'émetteur de lumière (24) est agencé avec son axe optique de manière sensiblement perpendiculaire à la surface plane au niveau de la paroi de tuyau (38) et
   le détecteur de lumière (28) est agencé au niveau de la paroi de tuyau (38) de manière adjacente à l'émetteur de lumière (24), dans lequel les axes optiques de l'émetteur de lumière (24) et du détecteur de lumière (28) forment un angle inférieur à 90°.

2. Détecteur selon la revendication 1, **caractérisé en ce que** deux surfaces (32, 34) ou les parois de tube (38, 40) sont agencées de manière plane et parallèle.

3. Détecteur selon la revendication 1, **caractérisé en ce que** les axes optiques de l'émetteur de lumière (24) et du détecteur de lumière (28) forment un angle de 35 à 55°, de préférence d'environ 45°.

4.  Détecteur selon l'une quelconque des revendications 1 à 3, **caractérisé en ce que** le point d'intersection des axes optiques de l'émetteur de lumière (24) et du détecteur de lumière (28) se trouve dans une zone qui s'étend de la limite du milieu entre la première paroi du tuyau (38) et le liquide (10) transporté dans le tuyau (18) jusqu'à 0,5 mm dans le liquide (10).

5.  Détecteur selon l'une quelconque des revendications 1 à 3, **caractérisé en ce que** le point d'intersection des axes optiques de l'émetteur de lumière (24) et du détecteur de lumière (28) se trouve au niveau de la limite du milieu entre la première paroi du tuyau (38) et le liquide (10) transporté dans le tuyau.

6.  Détecteur selon l'une quelconque des revendications 1 à 4, **caractérisé en ce qu'**une surface réfléchissante est prévue au niveau du côté (34, 40) du tuyau (10) opposé à l'émetteur de lumière (24) et au détecteur de lumière (26).

7.  Détecteur selon la revendication 6, **caractérisé en ce que** la surface réfléchissante est une surface de tuyau réfléchissante (40) ou une surface de boîtier réfléchissante (34).

8.  Détecteur selon l'une quelconque des revendications 1 à 7, **caractérisé en ce qu'**une vitre translucide (46) est agencée entre le tuyau (18) et l'émetteur de lumière (24) et le détecteur de lumière (26).

9.  Détecteur selon l'une quelconque des revendications 1 à 7, **caractérisé en ce que** l'émetteur de lumière (24) émet une lumière de longueur d'onde allant de 400 à 700 nm.

10. Détecteur selon l'une quelconque des revendications 1 à 8, **caractérisé en ce que** le détecteur de lumière (26) est une fibre optique ou une diode électroluminescente.

11. Détecteur selon l'une quelconque des revendications 1 à 10, **caractérisé en ce que** le détecteur de lumière (26) comprend un spectromètre relié à la fibre optique.

12. Détecteur selon l'une quelconque des revendications 1 à 11, **caractérisé en ce que** le détecteur de lumière (26) est relié en outre à une unité d'évaluation (70) pour test sanguin, dans lequel l'unité d'évaluation (70) est réalisée de telle sorte que l'unité d'évaluation calcule une fonction de modification de signal dépendant de la longueur d'onde à partir d'un signal de mesure dépendant de la longueur d'onde et d'un signal de référence également dépendant de la longueur d'onde, forme une intégrale de convolution à partir de la fonction de modification de signal sur une plage de longueur d'onde définie, et conclut à la présence de sang dans le liquide en raison de la valeur de l'intégrale de convolution.

13. Dispositif pour traiter le sang avec une unité de traitement de sang (62), un circuit sanguin (60) relié à l'unité de traitement de sang (62) et un circuit secondaire (66) également relié à l'unité de traitement de sang (62), en particulier un circuit de liquide de dialyse, dans lequel le circuit secondaire (66) comprend un système de tuyau et un détecteur (8) selon les revendications 1 à 12.

14. Procédé pour détecter du sang en solution, dans lequel un détecteur selon l'une quelconque des revendications 1 à 12 est utilisé, dans lequel une fonction de modification de signal dépendant de la longueur d'onde est calculée à partir d'un signal de mesure dépendant de la longueur d'onde et d'un signal de référence également dépendant de la longueur d'onde, et dans lequel une intégrale de convolution est formée à partir de la fonction de modification de signal et d'une fonction de convolution sur une plage de longueur d'onde définie et il est conclu à la présence de sang en raison de la valeur de l'intégrale de convolution.

15. Utilisation du détecteur (8) selon les revendications 1 à 12 pour la détection d'hémoglobine et ou de marqueurs, en particulier de bilirubine dans des liquides.

16. Utilisation du détecteur (8) selon les revendications 1 à 12 pour la détection de bulles d'air dans une solution optiquement dense.

45°

Fig 1

EP 2 032 967 B1

Fig 2

Fig.3 a

Fig.3b

Fig. 4.

Fig 5:

Fig. 6

Fig. 7

Fig. 8

EP 2 032 967 B1

Fig. 9

Fig. 10

18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0575712 A2
- EP 1083948 B1
- US 5644402 A
- US 6718190 B
- WO 2004057313 A
- US 5783826 A
- GB 1320533 A